# EUROPEAN PATENT SPECIFICATION

(11) **EP 0 353 662 B1**
(45) Date of publication and mention of the grant of the patent: **06.04.1994**
(21) Application number: 89114009.7
(22) Date of filing: 28.07.1989
(51) Int. Cl.: A01N 63/04, C12R 1/645, C12N 1/14

(54) **Use of ampelomyces quisqualis and a pure culture thereof**
Verwendung von Ampelomyces quisqualis und eine Reinkultur davon
Utilisation d'ampelomyces quisqualis et sa culture pure

(30) Priority: 02.08.1988 IL 87323
(43) Date of publication of application: 07.02.1990
(73) Proprietor: YISSUM RESEARCH DEVELOPMENT COMPANY OF THE HEBREW UNIVERSITY OF JERUSALEM, Jerusalem, 92 182 (IL)
(72) Inventor: Sztejnberg, Abraham, Rehovot 76 300 (IL)
(74) Representative: Grünecker, Kinkeldey, Stockmair & Schwanhäusser Anwaltssozietät

(56) References cited:
- PHYTOPARASITICA, vol. 16, February 1988, page 69, Priel Publisher, P0B 2385, Rehovot, 76120, IL; A. SZTEJNBERG et al.: "Ampelomyces Quisqualis for biological and integrated control of powdery mildew"
- BIOLOGICAL ABSTRACTS, vol. 75, no. 6, 1983, page 4548, column 1, abstract no. 44482, Philadelphia, PA, US; L. SUNDHEIM: "Control of cucumber powdery mildew by the hyperparasite Ampelomyces quisqualis and fungicides", & PLANT PATHOL. (LOND.) 31(3): 209-214. 1982
- BIOLOGICAL ABSTRACTS, vol. 68, no. 11, 1979, page 7018, column 2, abstract no. 69837, Philadelphia, PA, US; W.-D. PHILIP et al.: "Parasitism of Ampelomyces quisqualis on powdery mildew of cucumber and other vegetable species", & Z. PFLANZENKR. PFLANZENSCHUTZ, 86(3/4): 129-142, 1979

## Description

### FIELD OF INVENTION

The present invention is in the field of biological control of pests and more specifically concerns the biological control of powdery mildew (PMD), by a hyperparasite of the causative microorganism of PMD.

### BACKGROUND OF THE INVENTION AND PRIOR ART

In recent years there has been a growing interest in biological control of agricultural pests, i.e. by the use of microorganisms antagonistic to the pest, in view of the hazardous environmental effects of conventional chemical pesticides. Such a control has the advantage of being target specific and not polluting the environment.

PMD is a plant disease of world wide occurrence caused by various types of fungi which can infect many types of trees, flower plants, vegetables, fruit plants and various field crops, all of which will be referred to hereinafter collectively as "agricultural plants". A PMD infection is evidenced by a superficial, white or light grey powdery or felty growth on the surface of leaves, buds, young shoots, inflorescences, fruits and even flowers. In many cases the result of a PMD infection in agricultural plants is the reduction of the yields and quality of crop.

Up to date, no microorganisms which have the capability to combat PMD have been found. There has been a suggestion in the art (Wolf-Dieter Philipp and Gerd Cruger, Journal of Plant Diseases and Protection 86 (3/4), 129-142 (1979), to utilize a hyperparasite to the causative microorganisms of PMD, but all hyperparasites that have been isolated prior to the present invention, were found to have insufficient effectivity for the control of PMD in agricultural plants in the field in or in a greenhouse.

It is the object of the present invention to provide a pure culture of a hyperparasite of the causative microorganism of PMD, which may be utilised for controlling PMD.

### SUMMARY OF THE INVENTION

In accordance with the present invention, a novel fungal strain belonging to the fungal specie**s *Ampelomyces quisqualis*** has been isolated and termed AQ10. It was found that AQ10 is a hyperparasite of the causative fungi of PMD. It was further found in accordance with the invention that the conidia of AQ10 are potent anti-PMD agents that can be used effectively for combatting PMD in agricultural plants whereby a long felt want is satisfied for the first time.

Thus in accordance with the present invention, there is provided a pure culture of the strain AQ10 which was deposited in the culture collection of the Institut Pasteur (Collection Nationale de Cultures de Microorganismes - CNCM) on October 10, 1988, and accorded the designation I-807.

A pure culture of AQ10 may be subjected to a controlled mutagenic treatment, such as by various radioactive substances, chemical mutagens or by irradiation with γ- or X-rays, in order to obtain a mutant of the AQ10 strain whose conidia is also an antagonist of causative fungi of PMD and which may have some improved properties such as a higher potency, improved reproduction rate, improved susceptibility to storage and the like.

Conidia are the asexual reproductive spores of fungal microorganisms. The conidia of AQ10 are easily obtained in a substantially pure form from the AQ10 pure culture.

Thus, the present invention also provides an essentially pure aqueous suspension of AQ10 conidia.

Furthermore, the present invention provides an aqueous anti-PMD composition comprising an effective amount of conidia of AQ10 or of a mutant thereof, and, if desired, with phytologically acceptable additives compatible with said conidia.

Still further, the invention provides a method of controlling PMD in agricultural plants, comprising applying onto the plants an effective amount of conidia of AQ10 or of a mutant thereof. Such a treatment, which may be periodically repeated, is effective for destroying the causative microorganism of PMD, for inhibiting its proliferation as well as for preventing future PMD infestation.

The control of PMD in accordance with the invention may be effected in an open field or in a greenhouse. For prophylatic application, the conidia should be so formulated as to ensure their viability until onset of a PMD infestation whereupon they start immediately to exert their hyper-parasitic activity.

### A DETAILED DESCRIPTION OF THE INVENTION

### I Conservation and storage of the cultures.

The pure cultures of AQ10 may be conserved for some time in media which are suitable for fungal growth and proliferation. An example of such a medium is an agar substrate, which comprises wheat-bran extracts.

Due to the consumption of nutrition by AQ10 and the secretion of waste products, a sample of the culture comprising cells of AQ10 or conidia produced thereby should be transferred regularly into a new, fresh medium, e.g. once every three to four weeks. If the culture is growing on an agar substrate, conidia which are produced by the micro-organism can be harvested and then seeded onto a new agar substrate. Harvesting of the conidia is suitably performed by the addition of distilled water onto the agar substrate, and due to hypertonic pressure the ripe conidia are excreted into the supernatant fluid where they can easily be collected.

However, periodical transfer of conidia into new media may give rise to a mutagenic stress and in consequence a genetic drift of the culture may occur. Thus, such conservation of the culture is suitable mainly for short periods of time.

For storage periods of up to about 6 months, a mono-layer of the AQ10 cells on a solid matrix, such as BEA (bran extract agar), is kept at 4°C.

A suspension of AQ10 conidia in an aqueous solution comprising about 10⁷ conidia/ml, which is obtained by fermentation (see below), may be stored for longer periods, i.e. up to about 12 months. The conidia probably secrete antibiotic compounds which inhibit growth of other microorganisms, and in order for the inhibition to be effective a concentration of conidia of the order specified is required.

Long term storage may also be achieved by freeze drying the conidia. Such freeze dried conidia preserve their viability over prolonged storage periods.

### II Fermentation

Fermentation is required in order to obtain large quantities of AQ10, for the preparation of anti-PMD compositions. Fermentation may either be in liquid media such as potato broth (which is the soluble extract of boiled potatoes), corn-steep liquor (which is a syrup remaining after boiling of corn seeds in the starch industry), cotton meal extract (the extracted composites of cotton seed meal following boiling), whey, wheat-bran or a combination thereof, or may be carried out in a semi-solid medium, i.e. a wet particulate solid substrate, e.g. powdered carriers such as vermiculite and peat-moss or within wet granulated matrices such as of sorghum, wheat or barley grains.

Liquid fermentation is performed by inoculating a sample of AQ10 into a growth medium and aerobically incubating the medium for a sufficient amount of time. The optimal temperature for fermentation was found to be about 25°C and the optimal pH about 6.5 to about 8.0. Fermentation is preferably carried out under agitation e.g. in shaking flasks. Under such optimal conditions it is possible to obtain within seven to nine days a yield of about 1.5-2.0x10⁷ conidia/ml.

A suitable medium for performing the fermentation is potato broth. In this medium the yield of conidia after seven to nine days incubation is about 10 fold that of the other media listed above. However, it was found that a similar yield of conidia is achieved when utilising a whey medium supplemented with corn steep liquor with the relative volume of whey to corn steep liquor being about 2:1. Against this it was found in accordance with the invention that under certain conditions AQ10 grows and proliferates better in the dark. Therefore, in accordance with the invention fermentation of AQ10 is sometimes preferably carried out in the dark.

Semi-solid fermentations may be carried out on a number of carriers as specified above. The final number of conidia which can be harvested after such fermentation is higher than that achieved with liquid fermentation, although the fermentation time, which depends on the carrier type, is usually longer.

### III Formulation of anti-PMD compositions

AQ10 conidia are generally applied onto the agricultural plants by spraying an aqueous composition containing 10⁶ conidia/ml.

For effective treatment an aqueous composition of AQ10 conidia should be spread so as to cover the whole surface of the leaves and the other plant organs such as inflorescences, fruits or flowers, which are infected with PMD. When water is sprayed onto a waxy surface such as leaves, wetting is incomplete as drops tend to form. Consequently the aqueous anti-PMD compositions according to the invention preferably contain additives such as surfactants or wetting agents, e.g. , so-called thinners, which are typically added in an amount of about 0.2% w/w.

Since the anti-PMD AQ10 microorganism according to the invention is a hyperparasite of the causative microorganisms of PMD, no nutrients have to be incorporated in the compositions. However, at times it may be desirable to add such nutrients and also protectants to help keep the conidia viable, such as for example when the compositions are to be used for prophylactic treatment for which it is necessary that the micro-organisms will be viable for long periods of time.

### DESCRIPTION OF SOME SPECIFIC EMBODIMENTS

In the following description some specific embodiments will be described, it being understood that the invention is not limited thereto and that various modifications within the scope of the claims are possible.

### EXAMPLE 1: Culture of AQ10

AQ10 was incubated on plates which contained BEA substrate.

For the preparation of BEA, 100g of wheat-bran were dissolved in 1 litre of distilled water, and the solution was then sterilised in an autoclave for twenty minutes in order to extract the wheat bran and thereafter filtered through gauge filters, (20µm pores). Following autoclaving and subsequent filtration, water was supplemented to 1 litre, and 20g of malt extract, 2g of DL-asparagine and 20g of agar were admixed into the solution, which was then autoclaved and poured into agar plates and cooled.

After three weeks of incubation, 10ml distilled water was added to each plate and due to the hypertonic pressure, the ripe conidia were excreted into the supernatant where they were collected, the yield being 10⁷ to 10⁸ conidia/ml.

The harvested conidia were then either replated on fresh BEA plates, freeze dried and stored at 4°C or used for fermentation.

### EXAMPLE 2: Storage of AQ10

### (a) Storage of monolayers of AQ10:

AQ10 conidia were seeded onto a BEA plate, prepared as in Example 1, and incubated for 14 days until a monolayer of AQ10 was obtained. The agar plate was then stored at 4°C, with no loss of cell viability for at least six months.

### (b) Storage of conidial suspensions:

Seeding of AQ10 on BEA plates and harvesting of conidia were performed in accordance with Example 1. The conidial suspension containing 10⁷ to 10⁸ conidia/ml was then stored at 4°C for up to 12 months, with no apparent loss in their viability.

During this term no contamination by other fungi or by bacteria was observed, probably due to a secretion of antibiotic substances present in the conidia preparation.

### (c) Freeze drying of conidia:

A suspension of 10⁶ conidia/ml, was centrifuged at 12000g for 10 min. after which the resulting pellet was resuspended with 10% skimmed milk. Thereafter, the suspension was frozen in dry ice-acetone, lyophilized, and thereafter stored at -4°C. Rehydration was achieved by adding sterile water to the lyophilized conidia.

After three months there were essentially no signs of loss in viability when replated onto BEA plates.

### EXAMPLE 3: Fermentation

### (a) Liquid Fermentation in various growth media.

A sample of 100ml of conidia, containing about 10⁶ conidia/ml, was inoculated into flasks containing each 1 litre of a fermentation medium having a pH between 6.5-8.0. Each of the flasks contained a different medium. The incubation temperature was about 25°C. During incubation the flasks were shaken, and after nine days, the yield of conidia was determined. The results are in the following Table 1:

**Table 1**

| AQ10 | | |
|---|---|---|
| Growth medium | Yield of conidia (x10⁶/ml) | |
| | Average* | Maximal |
| a) 10 % Wheat-bran | 9.2 | 10.0 |
| b) 20 % Potato Extract | 12.9 | 17.0 |
| c) 20 % Whey | 2.1 | 3.9 |
| d) 20 % Whey + 7.5 g Cotton Meal Extract | 4.6 | 5.5 |
| e) 10 % Whey + 6 ml Corn Steep Liquor (CSL) | 10.6 | 13.0 |
| f) 20 % Whey + 14 ml CSL | 12.3 | 17.0 |
| g) 20 % Whey + 12 ml CSL | 16.0 | 19.0 |

| | | |
|---|---|---|
| * Four replicates | | |

It may be seen from the above results, that the highest yield with a medium of a single source is obtained in 20% potato extract. However a combination of 20% whey with CSL, gave an even higher yield.

### (b) The effect of light on AQ10 growth

A sample of a suspension of AQ10 conidia containing 10⁶ conidia/ml, was in one experiment seeded on a BEA substrate, contained in petri dishes or inoculated into a potato/dextrose (PD) liquid media and in another experiment inoculated into a liquid sucrose media. Some of the AQ10 cultures were either exposed to sunlight during daytime throughout the incubation, while others were darkened by covering them with aluminium foil. The temperature and pH were as above. After 10-14 days of incubation, the yield of conidia was determined, and the results are represented in the following Table 2:

**Table 2**

| Growth medium | Total No. of Conidia | |
|---|---|---|
| | **LIGHT** | **DARK** |
| Liquid sucrose | 1.6 x 10⁸ | 1.9 x 10⁹ |
| P D B | 3.0 x 10⁷ | 9.0 x 10⁸ |
| BEA/agar | 6.0 x 10⁵ | 3.0 x 10⁷ |

It is clear from the above results, that under the conditions described far better yields are achieved when AQ10 is grown in the dark.

### (c) Semi solid fermentation:

Sorghum grains were mixed with an AQ10 conidia suspension containing 10⁶ conidia/ml. The mixture was then incubated in a dark container.

After 20 days of incubation 6x10⁷ conidia/ml were obtained.

### EXAMPLE 4:: Formulation of AQ10 conidia

### (a) Field Tests

Marrow squash plants in the open field were infected with PMD. Plants in a 0.25 acre plot were divided into groups of about 40 plants each, each group receiving one of the following treatments:
1. Spraying of an AQ10 conidia aqueous suspension such as obtained by fermentation in accordance with Example 3(a) using Growth Medium b) of Table 1. Altogether four separate sprays were made, each carried out within five days from the previous one.
2. Spraying of each of the following conventional chemical fungicides: Afugan (Hoechst, FRG), Magen (Nippon Soda Company Ltd. Japan), San619 (Sandoz, Switzerland), Ofir (Ciba Geigy, Switzerland). In each case three separate sprays were carried out as in (1) above.
3. Spraying of Tilt (Ciba Geigy, Switzerland), which is a systemic fungicide. Altogether three sprays were carried out as in (1) above.
4. Untreated control

The squash was handpicked every two days and crops of the four groups were kept separately and was divided into first and second rate quality, and weighed. The results are summarized in the following Table 3:

**Table 3**

| Crop in Kg | | | | | | | |
|---|---|---|---|---|---|---|---|
| Quality rating | Untreated control | AQ₁₀ | Ophir | Afugan | Magen | San619 | Tilt |
| 1st quality | 90.3 | 113.7 | 119.6 | 134.5 | 125.3 | 128.7 | 151.5 |
| 2nd quality | 30.2 | 53.4 | 38.4 | 49.3 | 59.3 | 61.0 | 71.0 |
| Total: | 120.5^{c} | 167.1^{b} | 158.0^{b} | 183.0^{b} | 184.6^{b} | 189.7^{b} | 222.5^{a} |
| The letters against the Totals indicate statistic significance: All Totals marked with a "b" are not significantly different from one another. The Total marked with "a" is significantly different from the one marked with "c", and both are significantly different from the Totals marked with "b". | | | | | | | |

Despite the dry and hot weather, good hyperparasitism of AQ10 on PMD was observed throughout the season, as evidenced by microscopic observation of leaf samples.

Highest crop yield was obtained with tilt, which is a new chemical fungicide to which no resistance has as yet developed. AQ10 gave a crop yield similar to those obtained with the other fungicides, and significantly better than in the untreated control.

### (b) Control of PMD in mango trees.

Thirty six mango trees, in an organic farm in the Golan Heights, Northern Israel were divided into four groups each consisting of nine trees. Two groups of trees were treated with an AQ10 composition according to the invention, which was applied to each group with a different repetition rate. A third group served as an untreated control and the fourth was treated with sulphur, which is the conventional treatment against PMD in organic agriculture. The extent of PMD control was evaluated by counting the number of fruits as well as determining the average weight of total fruit per tree. The results are shown in the following Table 4:

**Table 4**

| Treatment | Average number of fruit/tree: | Average weight of fruit/tree (kg) |
|---|---|---|
| non-treated control | 26 ± 12^{a} | 12.50 ± 5.8^{a} |
| AQ10 every 5 days (6 wks) | 48 ± 12^{b} | 20.9 ± 4.9^{b} |
| AQ10 every 7 days (6 wks) | 45 ± 15^{b} | 19.32 ± 7.6^{b} |
| Sulphur treatment (as commonly used in organic plots) | 35 ± 15^{ab} | 15.72 ± 6.6^{ab} |
| The letters against the average numbers and average weight of fruits per tree indicate statistic significance: The numbers marked with "a" are significantly different from the numbers in the same column marked with "b"; the numbers marked with "ab" are not significantly different from the rest in the same column. | | |

The fruit yield from the AQ10 treatment is significantly higher than the non-treated control and is also superior to the conventional sulphur treatment.

## Claims (Claims for the following Contracting State(s): AT, BE, CH, DE, FR, GB, GR, IT, LI, LU, NL, SE)

1. A pure culture of the fungal strain Ampelomyces quisqualis AQ10 (CNCM. 1-807).

2. An essentially pure aqueous suspension of conidia of the fungal strain Ampelomyces quisqualis AQ10 (CNCM. 1-807).

3. A pure culture of a mutant of the fungal strain Ampelomyces quisqualis AQ10 (CNCM. 1-807), said mutant essentially retaining the anti-powdery mildew activity of the AQ10 strain.

4. An essentially pure aqueous suspension of conidia of a mutant according to claim 3.

5. An anti-powdery mildew aqueous composition containing 10⁶-10⁸ ml conidia/ml of suspension of the fungal strain Ampelomyces quisqualis AQ10 (CNCM. 1-807) of claim 1 or of a mutant of claim 3 and, if desired, at least one phytologically acceptable additive compatible with said conidia.

6. A composition according to claim 5, wherein said additive is a surfactant.

7. A method of controlling powdery mildew infestation and infection in agricultural plants in the open field and greenhouse, comprising applying to plants 10⁶-10⁸ ml/ml of suspension conidia of the fungal strain Ampelomyces quisqualis AQ10 (CNCM. 1-807) of claim 1 or a mutant of claim 3.

## Claims (Claims for the following Contracting State(s): ES)

1. A method of obtaining a pure culture of the fungal strain Ampelomyces quisqualis AQ10 (CNCM. 1-807) comprising isolating said fungal strain in a manner known per se.

2. A method of providing an essentially pure aqueous suspension of conidia of the fungal strain Ampelomyces quisqualis AQ10 (CNCM. 1-807) comprising suspending conidia obtained by fermentation of said fungal strain in a manner known per se.

3. A method of obtaining a pure culture of a mutant of the fungal strain Ampelomyces quisqualis AQ10 (CNCM. 1-807), said mutant essentially retaining the anti-powdery mildew activity of the AQ10 strain comprising subjecting the AQ 10 strain to controlled mutagenic treatment and isolating said mutant in a manner known per se.

4. A method of providing an essentially pure aqueous suspension of conidia of a mutant as obtained by the method of claim 3, comprising suspending conidia obtained by fermentation of said mutant in a manner known per se.

5. A method of providing an anti-powdery mildew aqueous composition containing 10⁶-10⁸ conidia/ml of suspension of the fungal strain Ampelomyces quisqualis AQ10 (CNCM.1-807) as obtained by the method of claim 1 or of a mutant as obtained by the method of claim 3 and, if desired, at least one phytologically acceptable additive compatible with said conidia comprising preparing said composition in a manner known per se.

6. The method according to claim 5, wherein said additive is a surfactant.

7. A method of controlling powdery mildew infestation and infection in agricultural plants in the open field and greenhouse, comprising applying to plants 10⁶-10⁸ conidia/ml of suspension of the fungal strain Ampelomyces quisqualis AQ 10(CNCM.1-807) as obtained by the method of claim 1 or a mutant as obtained by the method of claim 3.

## Patentansprüche (Patentansprüche für folgende(n) Vertragsstaat(en): AT, BE, CH, DE, FR, GB, ER, IT, LI, LU, NL, SE)

1. Reinkultur des Pilzstammes Ampelomyces quisqualis AQ10 (CNCM. 1-807).

2. Eine im wesentlichen reine, wäßrige Suspension von Conidien des Pilzstammes Ampelomyces quisqualis AQ10 (CNCM. 1-807).

3. Reinkultur einer Mutante des Pilzstammes Ampelomyces quisqualis AQ10 (CNCM. 1-807), wobei die Mutante die Wirksamkeit des AQ10-Stammes gegenüber Mehltau im wesentlichen noch besitzt.

4. Eine im wesentlichen reine, wäßrige Suspension von Conidien einer Mutante nach Anspruch 3.

5. Wäßrige Anti-Mehltau-Zusammensetzung, enthaltend 10⁶-10⁸ Conidien/ml Suspension des Pilzstammes Ampelomyces quisqualis AQ10 (CNCM. 1-807) nach Anspruch 1 oder einer Mutante nach Anspruch 3 und, wenn gewünscht, wenigstens ein phytologisch annehmbares Zusatzmittel, welches mit den Conidien verträglich ist.

6. Zusammensetzung nach Anspruch 5, worin das Zusatzmittel ein oberflächenaktives Mittel ist.

7. Verfahren zum Kontrollieren eines Mehltaubefalls und einer Infektion bei Nutzpflanzen im Freiland und Gewächshaus, umfassend das Anwenden von 10⁶-10⁸ Conidien/ml Suspension des Pilzstammes Ampelomyces quisqualis AQ10 (CNCM. 1-807) nach Anspruch 1 oder einer Mutante nach Anspruch 3 auf Pflanzen.

## Patentansprüche (Patentansprüche für folgende(n) Vertragsstaat(en): ES)

1. Verfahren zum Erhalten einer Reinkultur des Pilzstammes Ampelomyces quisqualis AQ10 (CNCM. 1-807), umfassend das Isolieren des Pilzstammes auf eine an sich bekannte Weise.

2. Verfahren zum Bereitstellen einer im wesentlichen reinen, wäßrigen Suspension von Conidien des Pilzstammes Ampelomyces quisqualis AQ10 (CNCM. 1-807), umfassend das Suspendieren von Conidien, erhalten durch Fermentation des Pilzstammes auf eine an sich bekannte Weise.

3. Verfahren zum Erhalten einer Reinkultur einer Mutante des Pilzstammes Ampelomyces quisqualis AQ10 (CNCM. 1-807), wobei die Mutante die Wirksamkeit des AQ10-Stammes gegenüber Mehltau im wesentlichen noch besitzt, umfassend das Aussetzen des AQ10-Stammes einer kontrollierten mutagenen Behandlung und das Isolieren der Mutante auf eine an sich bekannte Weise.

4. Verfahren zum Bereitstellen einer im wesentlichen reinen, wäßrigen Suspension von Conidien einer Mutante, erhalten durch das Verfahren nach Anspruch 3, umfassend das Suspendieren von Conidien, erhalten durch Fermentation der Mutante auf eine an sich bekannte Weise.

5. Verfahren zum Bereitstellen einer wäßrigen Anti-Mehltau-Zusammensetzung, enthaltend 10⁶-10⁸ Conidien/ml Suspension des Pilzstammes Ampelomyces quisqualis AQ10 (CNCM. 1-807), erhalten durch das Verfahren nach Anspruch 1 oder einer Mutante, erhalten durch das Verfahren nach Anspruch 3 und, wenn gewünscht, wenigstens ein phytologisch annehmbares Zusatzmittel, welches mit den Conidien verträglich ist, umfassend das Herstellen der Zusammensetzung auf eine an sich bekannte Weise.

6. Verfahren nach Anspruch 5, worin das Zusatzmittel ein oberflächenaktives Mittel ist.

7. Verfahren zum Kontrollieren eines Mehltaubefalls und einer Infektion bei Nutzpflanzen im Freiland und Gewächshaus, umfassend das Anwenden von 10⁶-10⁸ Conidien/ml Suspension des Pilzstammes Ampelomyces quisqualis AQ10 (CNCM. 1-807), erhalten durch das Verfahren nach Anspruch 1 oder einer Mutante, erhalten durch das Verfahren nach Anspruch 3, auf Pflanzen.

## Revendications (Revendications pour l'(les) Etat(s) contractant(s) suivant(s): AT, BE, CH, DE, FR, GB, GR, IT, LI, LU, NL, SE)

1. Culture pure de la souche de champignon Ampelomyces quisqualis AQ10 (CNCM. 1-807).

2. Suspension aqueuse sensiblement pure de conidies de la souche de champignon Ampelomyces quisqualis AQ10 (CNCM. 1-807).

3. Culture pure d'un mutant de la souche de champignon Ampelomyces quisqualis AQ10 (CNCM. 1-807), ledit mutant conservant essentiellement l'activité anti-oïdium de la souche AQ10.

4. Suspension aqueuse sensiblement pure de conidies d'un mutant selon la revendication 3.

5. Composition aqueuse anti-oïdium contenant 10⁶-10⁸ conidies/ml de suspension de la souche de champignon Ampelomyces quisqualis AQ10 (CNCM. 1-807) de la revendication 1 ou d'un mutant de la revendication 3 et, si on le désire, au moins un additif phytologiquement acceptable compatible avec lesdites conidies.

6. Composition selon la revendication 5, dans laquelle ledit additif est un agent tensio-actif.

7. Procédé pour lutter contre l'infestation et l'infection par l'oïdium dans les plantes cultivées à l'air libre et en serre, dans lequel on applique aux plantes 10⁶-10⁸ conidies/ml de suspension de la souche de champignon Ampelomyces quisqualis AQ10 (CNCM. 1-807) de la revendication 1 ou d'un mutant de la revendication 3.

## Revendications (Revendications pour l'(les) Etat(s) contractant(s) suivant(s): ES)

1. Procédé d'obtention d'une culture pure de la souche de champignon Ampelomyces quisqualis AQ10 (CNCM. 1-807) dans lequel on isole ladite souche de champignon de façon connue.

2. Procédé pour fournir une suspension aqueuse sensiblement pure de conidies de la souche de champignon Ampelomyces quisqualis AQ10 (CNCM. 1-807) dans lequel on met en suspension les conidies obtenues par fermentation de ladite couche de champignon de façon connue.

3. Procédé d 'obtention d'une culture pure d'un mutant de la souche de champignon Ampelomyces quisqualis AQ10 (CNCM. 1-807), ledit mutant conservant sensiblement l'activité anti-oïdium de la souche AQ10, dans lequel on soumet la souche AQ10 à un traitement de mutagenèse contrôlée et on isole ledit mutant de façon connue.

4. Procédé pour fournir une suspension aqueuse sensiblement pure de conidies d'un mutant obtenu par le procédé de la revendication 3, dans lequel on met en suspension des conidies obtenues par fermentation dudit mutant de façon connue.

5. Procédé pour fournir une composition aqueuse anti-oïdium contenant 10⁶-10⁸ conidies/ml de suspension de la souche de champignon Ampelomyces quisqualis AQ10 (CNCM. 1-807) obtenue par le procédé de la revendication 1 ou d'un mutant obtenu par le procédé de la revendication 3 et, si on le désire, au moins un additif phytologiquement acceptable compatible avec lesdites conidies, dans lequel on prépare ladite composition de façon connue.

6. Procédé selon la revendication 5, dans lequel ledit additif est un agent tensio-actif.

7. Procédé pour combattre l'infestation et l'infection par l'oïdium dans les plantes cultivées à l'air libre et en serre, dans lequel on applique aux plantes 10⁶-10⁸ conidies/ml de suspension de la souche de champignon Ampelomyces quisqualis AQ10 (CNCM. 1-807) obtenue par le procédé de la revendication 1 ou d'un mutant obtenu par le procédé de la revendication 3.
